# NEUE EUROPÄISCHE PATENTSCHRIFT

(11) **EP 0 265 854 B2**
(45) Veröffentlichungstag und Bekanntmachung des Hinweises auf die Entscheidung über den Einspruch: **24.03.1999**
(45) Hinweis auf die Patenterteilung: 03.04.1991
(21) Anmeldenummer: 87115530.5
(22) Anmeldetag: 23.10.1987
(51) Int. Cl.: C07C 47/55, C07C 45/63

(54) **Verfahren zur Herstellung von Fluorbenzaldehyden**
Process for preparing fluorobenzaldehydes
Procédé de préparation de fluorobenzaldéhydes

(30) Priorität: 31.10.1986 DE 3637156
(43) Veröffentlichungstag der Anmeldung: 04.05.1988
(73) Patentinhaber: Clariant GmbH, 65929 Frankfurt am Main (DE)
(72) Erfinder: Metz, Hans Joachim, Dr., D-6148 Heppenheim (DE); Warning, Klaus, Dr., D-6239 Eppstein/Taunus (DE)

(56) Entgegenhaltungen:
- EP-A- 61 113
- EP-A- 0 117 100
- EP-A- 0 164 619
- DE-A- 2 221 844
- DE-B- 2 039 426
- US-A- 4 579 976
- Fluoroorganic compounds in industry: applications and synthesis; Chemistry and Industry, 4.8.1986, S. 518-523
- Miller, Aromatic nucleophilic substituion, S. 79, 1968
- Weygand, Preparative Organic Chemistry, 1972, S. 209
- P.G.S. Field et al, Specialty Chemicals, Innovations in industrial synthesis and applications, Elsevier Applied Science, 1991, S. 64
- N.S.Isaacs, Physical organic chemistry. Longman Scientific & Technical, John Wiley & Sons, Inc., New York, US, 1987

## Beschreibung

Fluorbenzaldehyde sind Verbindungen, die sich vom Benzaldehyd C₆H₅CHO durch den Ersatz eines oder mehrerer H-Atome der C₆H₅-Gruppe durch F-Atome sowie gegebenenfalls auch noch durch andere Substituenten ableiten. Sie sind hauptsächlich Zwischenprodukte, z.B. bei der Herstellung von pharmazeutischen Präparaten und Pflanzenschutzmitteln.

Zur Herstellung der Fluorbenzaldehyde sind verschiedene Verfahren bekannt. So wird z.B. in der DE-AS 2 039 426 (Beispiel 7) die Herstellung von 2,4- und 3,4-Difluorbenzaldehyd in 70 bzw. 76 %iger Ausbeute beschrieben: die Umsetzung verläuft nach folgendem Formelschema:

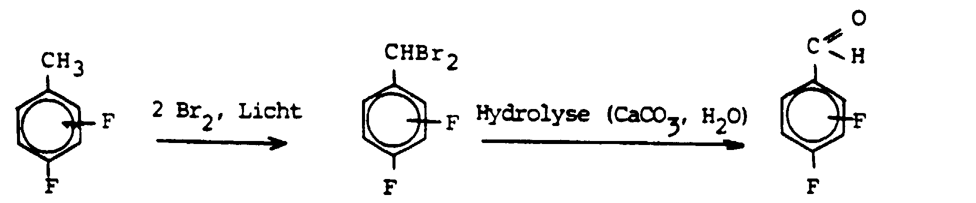

Zum 2,4-Difluorbenzaldehyd gelangt man nach EP-A-0 117 100 (Preparation 3, S. 16) in guter Ausbeute auch auf folgendem Wege: 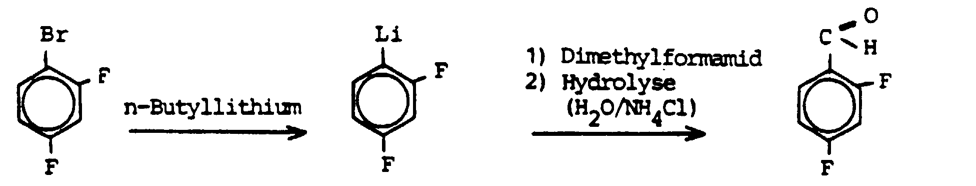 Die Ausgangsprodukte für diese Reaktionen sind z.B. durch ein- oder mehrfache Balz-Schiemann-Reaktion erhältlich. Unter der Balz-Schiemann-Reaktion versteht man die thermische Zersetzung von aromatischen Diazonium-tetrafluoroboraten zu den entsprechenden aromatischen Fluorverbindungen. Das Reaktionsprinzip, z.B. für die Herstellung von 2,4-Difluortoluol, läßt sich wie folgt wiedergeben: 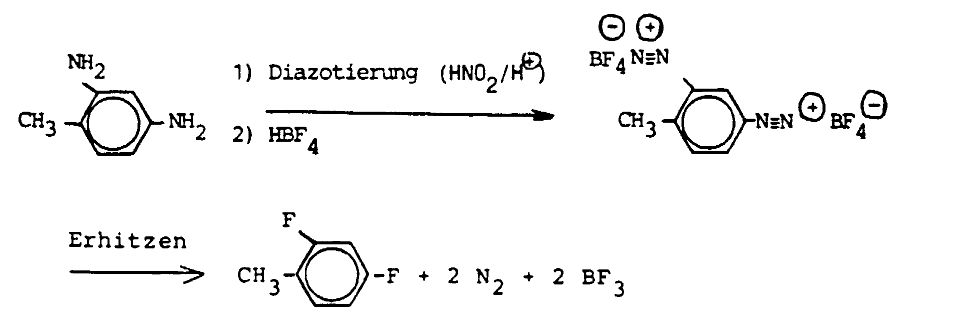 Ausgehend von gängigen Ausgangsprodukten hat man es demzufolge mit mehrstufigen Synthesen zu tun, deren Hydrolysestufen zur Bildung von salzhaltigen und daher umweltbelastenden Abwässern führen. Zudem bedarf die Herstellung mittels n-Butyllithium - wegen dessen Selbstentzündlichkeit an der Luftbesonderer Sicherheitsmaßnahmen.

Eine andere Herstellungsmethode für Fluorbenzaldehyde ist Gegenstand der EP-A-0 164 619. Hiernach werden Chlorbenzonitrile oder Chlorbenzoylhalogenide in einem dipolar-aprotischen Lösungsmittel, insbesondere Sulfolan (= Tetrahydrothiophen-S,S-dioxid), bei erhöhter Temperatur mit KF umgesetzt und das erhaltene Fluorbenzonitril bzw Fluorbenzoylhalogenid gegebenenfalls in an sich bekannter Weise in andere aromatische Fluorverbindungen, u.a. auch in Fluorbenzaldehyd übergeführt. Demnach kommt man zum 2,4,5-Trifluorbenzaldehyd, indem man zunächst das 2,4,5-Trichlorbenzonitril mit 3 KF zum 2,4,5-Trifluorbenzonitril umsetzt und dann wie folgt vorgeht, wobei der angegebene Weg vom fluorierten Nitril zum Aldehyd der auf S. 6 der EP-A beschriebenen Reaktionsfolge entspricht:

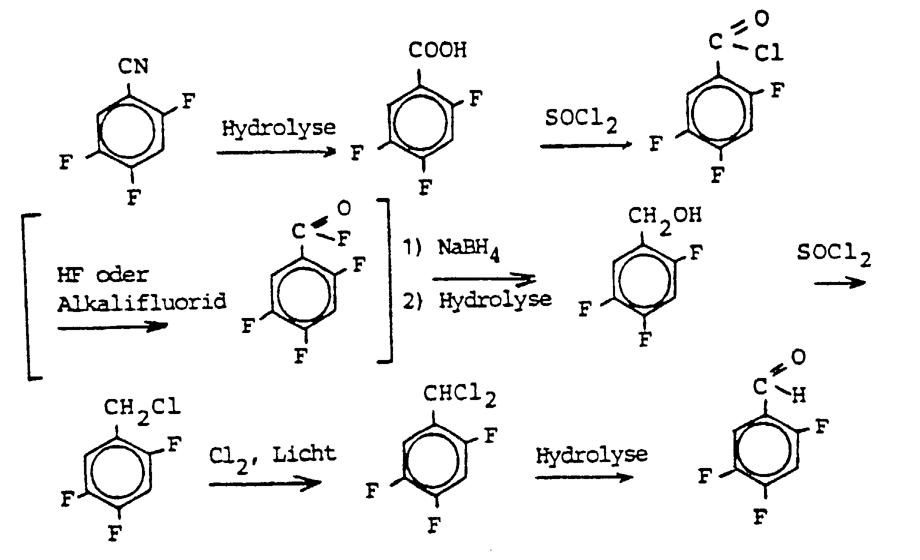

Wie ersichtlich, erfordert diese Synthese eine Vielzahl von Reaktionsschritten.

In dem Bestreben, eine einfachere Herstellungsmethode für Fluorbenzaldehyde bereitzustellen, wurde nun gefunden, daß dieses Ziel durch Umsetzung von Chlorbenzaldehyden mit Alkalifluoriden in einem aprotischen Reaktionsmedium erreicht wird. Erfindungsgegenstand ist daher ein Verfahren zur Herstellung von Fluorbenzaldehyden unter Anwendung der Chlor-Fluor-Austrauschreaktion an aromatischen Verbindungen mit Alkalifluorid in dipolar aprotischem Medium; das Verfahren ist dadurch gekennzeichnet, daß man für diese Chlor-Fluor-Austrauschreaktion Chlorbenzaldehyde einsetzt. Die Aldehydausbeuten liegen hierbei normalerweise um etwa 70 % und darüber. Mit dem Verfahren steht, ausgehend von gängigen Ausgangsprodukten, eine einfache, und einstufige Synthesemethode für Fluorbenzaldehyde zur Verfügung, bei der kein Abwasser entsteht.

Das Gelingen dieser Umsetzung ist außerordentlich überraschend, weil man nach der vorerwähnten EP-A-0 164 619 davon ausgehen mußte, daß Fluorbenzaldehyde nicht in einer einzigen Reaktionsstufe aus den entsprechenden Chlorbenzaldehyden durch Chlor-Fluor-Austausch mittels Alkalifluorid in aprotischem Medium hergestellt werden können, sondern daß man Fluorbenzaldehyde nur aus solchen Chloraromaten, welche keine CHO-Gruppe besitzen, auf ziemlich aufwendige Weise in mehreren Reaktionsschritten herstellen kann. Dies scheint auch verständlich, weil Alkalifluoride in aprotischem Medium, wo im Gegensatz zu wäßrigen Systemen praktisch keine Solvatation des Fluorids eintritt, als starke Basen bekannt sind - vgl. die Übersicht von G.G. Yakobson und N.E. Akhmetova in Synthesis 1983, S. 169 und 170 ("... alkali metal fluorides are rather strong bases..." vgl. S. 169, linke Spalte Abs. 1 und S. 170, Abs. 2 "The basic properties of alkali metal fluorides were first revealed in dehydrohalogenation reactions"). Gegenüber starken Basen sind aber bekanntlich Aldehyde kaum stabil. Eine Reaktion von Aldehyden mit Basen ist z.B. die sogenannte Cannizzaro-Reaktion (Oxidoreduktion von Aldehyden in Gegenwart von Basen). Auch eine Umsetzung mit den aktivierten H-Atomen in α-Stellung zur CO-, SO- oder SO₂-Gruppe der verwendeten aprotischen Lösungsmittel ist denkbar.

Zur Durchführung der Reaktion werden Chlorbenzaldehyde der Formel (I) eingesetzt. 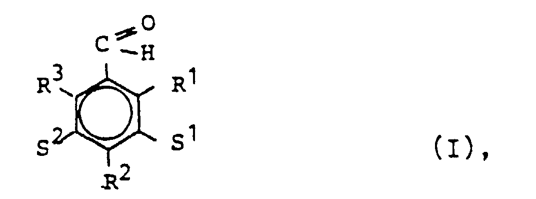 worin R¹, R² und R³ unabhängig voneinander H,F und/oder Cl darstellen, jedoch mindestens einer der Reste Chlor und vorzugsweise mindestens einer der Reste R¹ und R³ Wasserstoff ist und S¹ und S² Wasserstoff bedeuten.

Beispielhafte Ausgangsverbindungen für die erfindungsgemäße Umsetzung sind also o-, und p- Chlorbenzaldehyd, 2,4-Dichlor-, 2,4,6-Trichlorbenzaldehyd, 2-Chlor-4-fluor- und 2-Fluor-4-chlorbenzaldehyd, wobei die letzteren auch im Gemisch verwendet werden können, zumal sie zu einem einheitlichen Endprodukt führen. Besonders bevorzugte Ausgangsverbindung ist 2,4-Dichlorbenzaldehyd.

Für den Austausch der Chloratome in der Ausgangsverbindung ist stöchiometrisch 1 Mol Alkalifluorid pro Chloratom nötig. Wenn alle Chloratome des Ausgangschlorbenzaldehyds ausgetauscht werden sollen, ist es vorteilhaft, bis zu etwa 200 %, insbesondere etwa 100-120 % der stöchiometrischen Menge an Alkalifluorid einzusetzen. Der Einsatz von größeren Alkalifluoridmengen ist möglich, bietet aber keinen besonderen Vorteil.

Im Falle von Benzaldehyden mit mehreren Chloratomen läßt sich die Reaktion so durchführen, daß überwiegend partiell fluoriertes Produkt ensteht, indem man eine geringere Menge Alkalifluorid, als sie zum Austausch aller Chloratome benötigt wird, bei der Reaktion einsetzt.

Als Alkalifluorid werden z.B. Natriumfluorid oder Alkalifluoride für sich oder im Gemisch mit den einfachen Alkalifluoriden verwendet. Bevorzugt ist aber Kaliumfluorid oder eine Mischung desselben mit Rubidium- und/oder Caesiumfluorid in beliebigem Molverhältnis; besonders bevorzugt ist eine Mischung mit nur geringem (katalytischem) Anteil Rubidium-und/oder Caesiumfluorid (bis zu etwa 5 Mol %)

Als Lösungsmittel kommen für die Reaktion im Prinzip alle möglichen dipolar-aprotischen Lösungsmittel in Frage, vorzugsweise tertiäre Carbonsäureamide wie Dimethylformamid, Dimethylacetamid, N-Methylpyrrolidon, N, N, N'N',-Tetramethylharnstoff, N,N'-Dimethylimidazolidinon-2, Sulfoxyde wie Dimethylsulfoxyd und Sulfone wie Dimethylsulfon, Diphenylsulfon und das Sulfolan. Besonders bevorzugt sind das N-Methylpyrrolidon, das Dimethylsulfoxyd und das Dimethylsulfon, insbesondere aber Sulfolan. Die Lösungsmittel können sowohl einzeln als auch in Mischung miteinander eingesetzt werden.

Die Anfangskonzentration des Aldehyds kann in einem weiten Bereich liegen; vorzugsweise liegt sie bei etwa 0,5-3 mol/kg Lösungsmittel, insbesondere bei etwa 1-1,7 mol/kg dipolaraprotischem Medium (Lösungsmittel).

Die Umsetzung kann in einem weiten Temperaturbereich ablaufen. Man arbeitet zweckmäßig bei Temperaturen zwischen etwa 180 und 230°C.

Die erfindungsgemäße Reaktion wird vorzugsweise so durchgeführt, daß der Ausgangschlorbenzaldehyd mit dem Lösungsmittel und dem Alkalifluorid über längere Zeit unter Rühren und Schutzgasatmosphäre, z.B. Argon, vorzugsweise aber Stickstoff, erhitzt wird. Der Ablauf der Reaktion wird z.B. gaschromatographisch überwacht Nach Erreichen des gewünschten Umsetzungsgrades erfolgt die Aufarbeitung des Produkts: Vorzugsweise durch Abfitrieren unter Inertgasatmosphäre wird der anorganische Teil (Alkalihalogenid) abgetrennt. Anschließend wird das in der organischen Phase befindliche Produkt gereinigt, vorzugsweise durch Rektifikation unter vermindertem Druck.

Die Erfindung wird durch die nachstehenden Beispiele näher erläutert, wobei alle Operationen unter N₂ als Schutzgas durchgeführt wurden.

### Beispiele

1) 175 g (1 mol) 2,4-Dichlorbenzaldehyd wurden zusammen mit 1000 g Sulfolan und 151 g (2,6 mol) Kaliumfluorid 15 h auf 210-215°C erhitzt. Das Reaktionsgemisch wurde während der gesamten Reaktionsdauer durch ein kräftiges Rührwerk durchmischt, um ein Absetzen der unlöslichen Salze zu vermeiden. Anschließend kühlte man auf Raumtemperatur und trennte die anorganischen Anteile mittels einer mit Stickstoff überlagerten Nutsche ab. Es wurde zweimal mit je 100 g frischem Sulfolan nachgewaschen. Aus dem Filtrat erhielt man durch Rektifikation unter vermindertem Druck an einer Kolonne mit ca. 20 theor. Böden 96,6 g = 68 % d.Th. 2,4-Difluorbenzaldhyd vom Siedepunkt 70,5 °C/40 mbar. Erstarrungspunkt: ca. 2°C
2) 56 g (0,4 Mol) 4-Chlorbenzaldehyd wurden zusammen mit 200 g 1,3-Dimethylimidazolidin-on-2, 29,5 g Kaliumfluorid und 4,03 g Cäsiumfluorid (5 mol-%, bezogen auf Kaliumfluorid) 20 h unter kräftigem Rühren auf 215°C erhitzt. Durch Gaschromatographie wurden im Reaktionsgemisch 12,7 Gew.-% (= 32,4 g) 4-Fluorbenzaldehyd entsprechend 65,2 % und 4,3 Gew.-% (= 11,0 g) 4-Chlorbenzaldehyd entsprechend 19,6 %, jeweils auf das Ausgangsmaterial bezogen, nachgewiesen. Die Aufarbeitung erfolgt wie im Beispiel 1 beschrieben.
3) 158,5 g (1 mol) eines Gemisches aus 56 % 4-Chlor-2-fluorbenzaldehyd und 44 % 2-Chlor-4-fluorbenzaldehyd wurden zusammen mit 700 g Sulfolan und 75 g (1.29 mol) Kaliumfluorid 10 h auf 215°C erhitzt. Die Aufarbeitung erfolgte wie im Beispiel 1. Man erhielt 99,5 g 2,4-Difluorbenzaldehyd entsprechend 70 % v. Einsatz.

## Patentansprüche

1. Verfahren zur Herstellung von Fluorbenzaldehyden, dadurch gekennzeichnet. daß man Chlorbenzaldehyde der Formel (I) 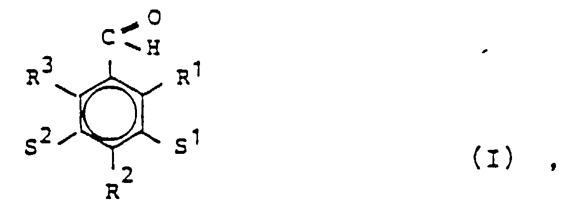 worin R¹, R² und R³ unabhängig voneinander H, F und/oder Cl darstellen, jedoch mincestens einer der Reste Chlor und vorzugsweise mindestens einer der Reste R¹ und R³ Wasserstoff ist und S¹ und S² Wasserstoff sind, einsetzt, und diese einer Chlor-Fluor-Austausch-Reaktion mit Alkalifluorid in dipolar-aprotischem Medium unterwirft.

2. Verfahren nach Anspruch 1, dadurch gekennzeichnet, daß man als Chlorbenzaldehyd 2,4-Dichlorbenzaldehyd einsetzt.

3. Verfahren nach einem oder mehreren der Ansprüche 1 - 2, dadurch gekennzeichnet, daß man für den Austausch aller Chloratome des Chlorbenzaldehyds bis zu etwa 200 %, bevorzugt etva 100 - 120 % der stöchiometrischen Menge an Alkalifluorid einsetzt.

4. Verfahren nach einem oder mehreren der Ansprüche 1 - 2, dadurch gekennzeichnet, daß man für den Austausch nur eines Teils der Chloratome eine geringere Menge Alkalifluorid einsetzt als zum Austausch aller Chloratome benötigt wird.

5. Verfahren nach einem oder mehreren der Ansprüche 1 - 4, dadurch gekennzeichnet, daß als Alkalifluorid Kaliumfluorid oder Mischungen desselben mit Rubidium- und/oder Cäsiumfluorid eingesetzt werden, wobei die Menge des Rubidium- und/oder Cäsiumfluorids bevorzugt höchstens 5 Mol-% beträgt.

6. Verfahren nach einem oder mehreren der Ansprüche 1 - 5, dadurch gekennzeichnet, daß man als dipolar-aprotische Medium tertiäre Carbonsäureamide, Dimethylsulfoxyd und/oder Dimethylsulfon, insbesondere Sulfolan, einsetzt.

7. Verfahren nach einem oder mehreren der Ansprüche 1 - 6, dadurch gekennzeichnet, daß man die Umsetzung bei Temperaturen zwischen etwa 180 und 230°C durchführt.

8. Verfahren nach einem oder mehreren der Ansprüche 1 - 7, dadurch gekennzeichnet, daß die Anfangskonzentration des Aldehyds im Bereich von etwa 0,5 bis 3, vorzugsweise 1 bis 1,7 mol/kg dipolar-aprotischem Medium liegt.

9. Verfahren nach einem oder mehreren der Ansprüche 1 - 8, dadurch gekennzeichnet, daß man unter Schutzgas arbeitet.

## Claims

1. A process for the preparation of fluorobenzaldehydes, which comprises employing chlorobenzaldehydes of the formula (I) 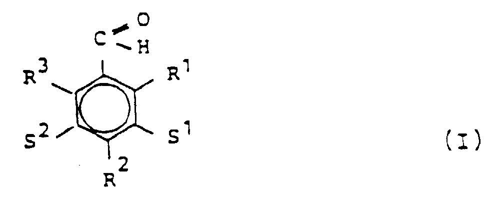 in which R¹, R² and R³ are independently of each other H, F and/or Cl, but at least one of the radicals is chlorine and preferably at least one of the radicals R¹ and R³ is hydrogen, and S¹ and S² are hydrogen and subjecting these to a chlorine-fluorine exchange reaction with alkali metal fluoride in a dipolar aprotic medium.

2. The process as claimed in claim 1, wherein 2,4-dichlorobenzaldehyde is employed as the chlorobenzaldehyde.

3. The process as claimed in one or more of claims 1 - 2, wherein up to about 200%, preferably about 100 - 120%, of the stoichiometric amount of alkali metal fluoride is employed for the exchange of all the chlorine atoms of the chlorobenzaldehyde.

4. The process as claimed in one or more of claims 1 - 2, wherein a smaller amount of alkali metal fluoride is employed for the exchange of only a part of the chlorine atoms than is needed for the exchange of all the chlorine atoms.

5. The process as claimed in one or more of claims 1 - 4, wherein potassium fluoride or mixtures thereof with rubidium fluoride and/or cesium fluoride are employed as the alkali metal fluoride, the amount of rubidium fluoride and/or cesium fluoride preferably being at most 5 mol%.

6. The process as claimed in one or more of claims 1 - 5, wherein tertiary carboxamides, dimethyl sulfoxide and/or dimethyl sulfone, in particular sulfolane, are employed as the dipolar aprotic medium.

7. The process as claimed in one or more of claims 1 - 6, wherein the reaction is carried out at temperatures between about 180 and 230°C.

8. The process as claimed in one or more of claims 1 - 7, wherein the starting concentration of the aldehyde is in the range from about 0.5 to 3, preferably 1 to 1.7, mol/kg of dipolar aprotic medium.

9. The process as claimed in one or more of claims 1 - 8, which is carried out under protective gas.

## Revendications

1. Procédé de préparation de fluorobenzaldéhydes, caractérisé en ce qu'on utilise, comme chlorobenzaldéhydes, des composés répondant à la formule I : 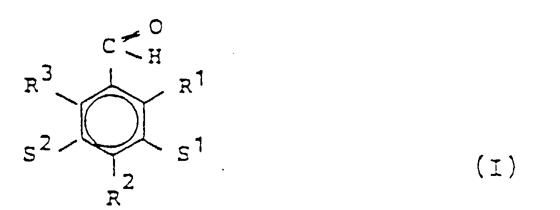 dans laquelle R¹, R² et R³ représentent chacun, indépendamment les uns des autres, H, F ou Cl, mais au moins un de ces symboles représentant le chlore, et, de préférence, au moins un des symboles R¹ et R³ représentant l'hydrogène, et S¹ et S² représentent l'hydrogène et on les soumet à une réaction d'échange chlore/fluor avec un fluorure de métal alcalin en milieu dipolaire aprotique.

2. Procédé selon la revendication 1, caractérisé en qu'on utilise le dichloro-2,4 benzaldéhyde comme chlorobenzaldéhyde.

3. Procédé selon une ou plusieurs des revendications 1 à 2, caractérisé en ce qu'on utilise, pour l'échange de tous les atomes de chlore du chlorobenzaldéhyde, jusqu'à environ 200 %, de préférence d'environ 100 à 120 %, de la quantité stoechiométrique du fluorure de métal alcalin.

4. Procédé selon une ou plusieurs des revendications 1 à 2, caractérisé en ce qu'on utilise, pour l'échange de seulement d'une partie des atomes de chlore, une quantité du fluorure de métal alcalin plus faible que celle qui est nécessaire pour l'échange de tous les atomes de chlore.

5. Procédé selon l'une quelconque des revendications 1 à 4, caractérisé en ce qu'on utilise, comme fluorure d'un métal alcalin, le fluorure de potassium ou des mélanges de celui-ci avec le fluorure de rubidium et/ou le fluorure de césium, la quantité de fluorure de rubidium et/ou de césium étant de préférence d'au plus 5 % en moles.

6. Procédé selon l'une quelconque des revendications 1 à 5, caractérisé en ce qu'on utilise, comme milieu aprotique dipolaire, un carboxamide tertiaire, le diméthylsulfoxyde et/ou la diméthylsulfone, plus particulièrement le sulfolan.

7. Procédé selon l'une quelconque des revendications 1 à 6, caractérisé en ce qu'on effectue la réaction à des températures comprises entre environ 180 et 230°.

8. Procédé selon l'une quelconque des revendications 1 à 7, caractérisé en ce que la concentration initiale de l'aldéhyde est comprise entre 0,5 et 3 mol, de préférence entre 1 et 1,7 mol, par kilogramme du milieu aprotique dipolaire.

9. Procédé selon l'une quelconque des revendications 1 à 8, caractérisé en ce que l'on opère sous un gaz protecteur.
